# EUROPEAN PATENT APPLICATION

(11) **EP 2 221 082 A1**
(43) Date of publication of application: **25.08.2010**
(21) Application number: 09172387.4
(22) Date of filing: 07.10.2009
(51) Int. Cl.: A61M 25/10, A61B 17/12

(54) **A catheter system for preventing and treating restenosis in coronary and peripheral arteries, in a single vessel or bifurcation**

(30) Priority: 20.02.2009 BR 09006222
(71) Applicant: BRZ Biotecnologia Ltda, 90480-000 Porto Alegre RS (BR)
(72) Inventor: Zago, Alexandre Do Canto, 90450-050, Porto Alegre (BR); Ferlin, Elton Luiz, 92410-450, Canoas (BR); Rossi, Rodrigo, 95034-190, Caxias do Sul (BR); Raudales, José Jesus Casco, 90640-040, Porto Alegre (BR); Rodrigues, Luiz Fernando Junior, 91140-001, Porto Alegre (BR); Bortolini, Marco Aurelio Ghilosso, 90460-080, Porto Alegre (BR); Zago, Alcides José, 90510-040, Port Alegre (BR)
(74) Representative: Schweiger, Martin

(57) **Abstract**

A catheter system for preventing and treating restenosis in coronary or peripheral arteries, in a single vessel or in a bifurcation, which comprises, at least, one occlusion-infusion catheter (10) fitted with a balloon (11) disposed in the distal portion and inflatable through a lumen (12) disposed in the proximal portion of said occlusion-infusion catheter (10), the surface of said occlusion-infusion catheter (10) having perforations intended for inserting and sliding out the guide-wire (13), and one or more perforations (14) on the distal end of the balloon (11) to release the drug; and, at least, one occlusion catheter (20) fitted with a balloon (21) disposed in the distal portion and inflatable through a lumen (22) disposed in the proximal portion of said of said occlusion catheter (20), and perforations on the surface intended for inserting and sliding out the guide-wire (23), is described

## Description

### FIELD OF THE INVENTION:

This invention refers to a catheter system for preventing and treating restenosis in coronary and peripheral arteries, in a single vessel or in a bifurcation. More specifically, it comprises a catheter system for preventing and treating intra-stent restenotic lesions, which comprises, at least, one occlusion-infusion catheter to staunch the blood flow and, at the same time, to administer drugs, and, at least, one occlusion catheter to retain the drug injected into the arterial portion to be treated.

### BACKGROUND OF THE INVENTION:

The lesion of the wall of a blood vessel damages many vessel components, leading to the loss of endothelial cells, rupture (dissection) of the intima and the media of the vessel (endothelial and subendothelial tissue), and inflammation. The response of the vessel to these three effects is largely responsible for neointima formation and restenosis.

Restenosis is a narrowing of an artery caused by the abnormal growth of the vessel wall in reaction to the inflation of a balloon or implantation of a stent under high pressure.

The balloon dilation of the occluded arterial lesions increases the arterial lumen diameter in order to restore the proper tissue perfusion. After defining the site of arterial puncture, the artery is catheterized, the patient being anticoagulated. Then the balloon catheter is inserted over the guide wire. The balloon is inflated and/or the stent is implanted at the site of stenosis.

The request for invention registration BR0415518 describes a catheter system and a method for implanting a stent in a vessel at the level of a bifurcation or a vessel ramification, said catheter system comprising a first balloon catheter, a second balloon catheter, and a release connection device to keep both first and second balloon catheters disposed next to each other or aligned with each other along the longitudinal axis. The connection system allows moving the catheter system of a unit forward and helps prevent the stent of the catheters from shifting prematurely or inadvertently, which is, however, releasable in such a manner that one or both balloon catheters may be released from the connection device and maneuvered separately from the rest of the catheter system whenever desired. The method uses a modified technique of "simultaneously inflated balloons" to install the described catheter system in a bifurcation of vessels.

The technical literature reveals balloon catheters that deliver drugs into the wall of an artery or a vessel, sometimes, making the drug diffuse into the proximal and distal portions of the balloon, which may lead to the formation of blood clots and infusion of doses higher than necessary with the ensuing adverse effects.

Thus, a catheter system for preventing and treating restenosis in coronary or peripheral arteries, in a single vessel or in a bifurcation, which can allow using, at least, one occlusion-infusion catheter to staunch the blood flow and to deliver the drug into the artery or vessel wall, and an occlusion catheter to temporarily retain the drug in a compartment created in the artery during the inflation of both catheters, such catheter system for preventing and treating restenosis in coronary and peripheral arteries, in a single vessel or in a bifurcation being described and claimed herein.

### SUMMARY

In general, this invention refers to a catheter system for preventing and treating restenosis in coronary and peripheral arteries, in a single vessel or in a bifurcation, which comprises, at least one occlusion-infusion catheter to staunch the blood flow and, at the same time, to deliver drugs, and, at least one occlusion catheter to retain the drug injected into the arterial portion to be treated.

The invention is characterized by a catheter system for preventing and treating restenosis in coronary and peripheral arteries, in a single vessel or in a bifurcation, which aims at preventing and/or treating post-angioplasty restenosis with a balloon catheter or post stent implantation in coronary or peripheral arteries.

The invention is characterized by a catheter system for preventing and treating restenosis in coronary or peripheral arteries, in a single vessel or in a bifurcation, which prevents and treats intra-stent restenotic lesions in arterial bifurcations where the restenosis rates are higher, by using, at least, one occlusion-infusion catheter and, at least, two occlusion catheters, one of them being positioned in the main vessel and the other in the lateral branch.

The invention is characterized by a catheter system for preventing and treating restenosis in coronary or peripheral arteries, in a single vessel or in a bifurcation, which potentializes the penetration of the drug into the artery wall in order to inhibit the neointimal hyperproliferation.

### BRIEF DESCRIPTION OF THE DRAWINGS

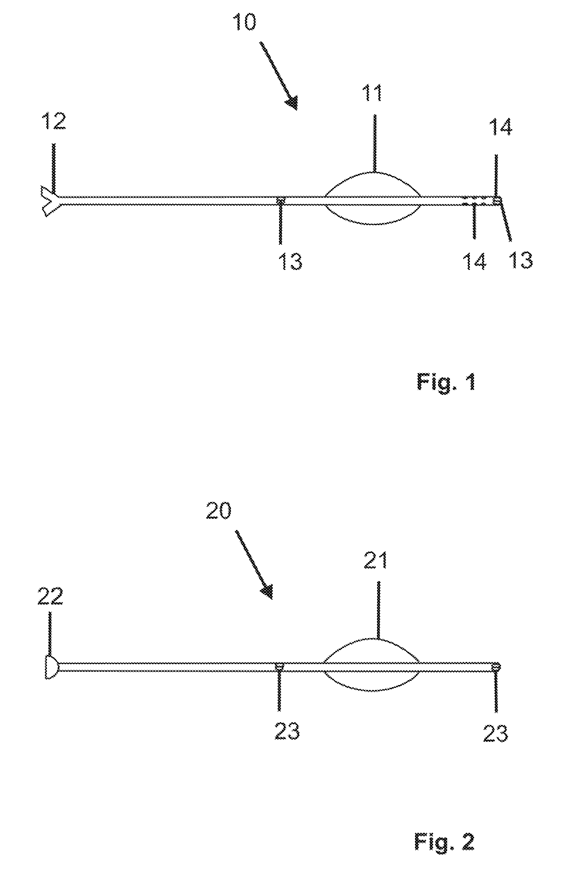

Figure 1 is an occlusion-infusion catheter.
Figure 2 is an occlusion catheter.
Figure 3 - an arrangement of the infusion-occlusion catheter and occlusion catheter system in a coronary artery.
Figure 4 - is an arrangement of the system consisting of an occlusion-infusion catheter and two occlusion catheters in an arterial bifurcation.

### DETAILED DESCRIPTION OF THE INVENTION

The catheter system for preventing and treating restenosis in coronary and peripheral arteries, in a single vessel or in a bifurcation, subject matter of this invention, comprises, at least, one occlusion-infusion catheter (10) fitted with a balloon (11) which, when inflated, stanches the blood flow and, at the same time, delivers a drug into the artery or vessel wall, and, at least, one occlusion catheter (20) fitted with a balloon (21), which, when inflated, retains the drug injected in the arterial portion (100) to be treated.

The occlusion-infusion catheter (10) has a balloon (11), preferably, a complacent one, said balloon (11) being disposed in the distal portion and inflatable through a lumen (12) disposed in the proximal portion of said occlusion-infusion catheter (10), the surface of said occlusion-infusion catheter (10) having perforations intended for inserting and sliding out the guide-wire (13), and one or more perforations at the distal end of the balloon intended for releasing the drug.

The occlusion catheter (20) has a balloon (21), preferably, a complacent one, said balloon (21) being disposed in the distal portion and inflatable through a lumen (22) disposed in the proximal portion of said occlusion balloon (20), and perforations on the surface intended for inserting and sliding out the guide-wire (23).

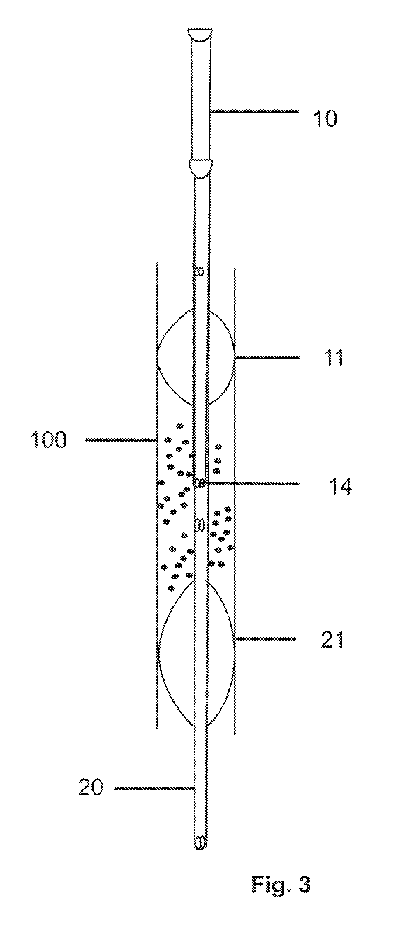

By means of examples, in such situations as prevention and treatment of restenosis in coronary and peripheral arteries in a single vessel, as shown in Figure 3, an occlusion catheter (2) is inserted in the artery or in the vessel (100) and then the occlusion-infusion catheter (10) is inserted and proximally positioned. Next, the balloon (11) of the occlusion-infusion catheter (10) is inflated, closing the path (100) and avoiding the blood flow passage. Later on, saline solution is applied to wash the artery and then the drug is infused. During the infusion of the drug, the occlusion catheter (20) is inflated in order to retain the drug inside the arterial portion (100), which is being treated, and in such a manner that a compartment is created. The drug is released through the perforations (14) disposed on the distal surface of the occlusion-infusion catheter (10), the drug being limited to the area delimited by the balloons of the catheters (10 and 20) and penetrating the artery or vessel wall (100) without diffusing it into the blood circulation. After the procedure has been finished, the balloons (11 and 21) are deflated and the catheters (10 and 20) are removed.

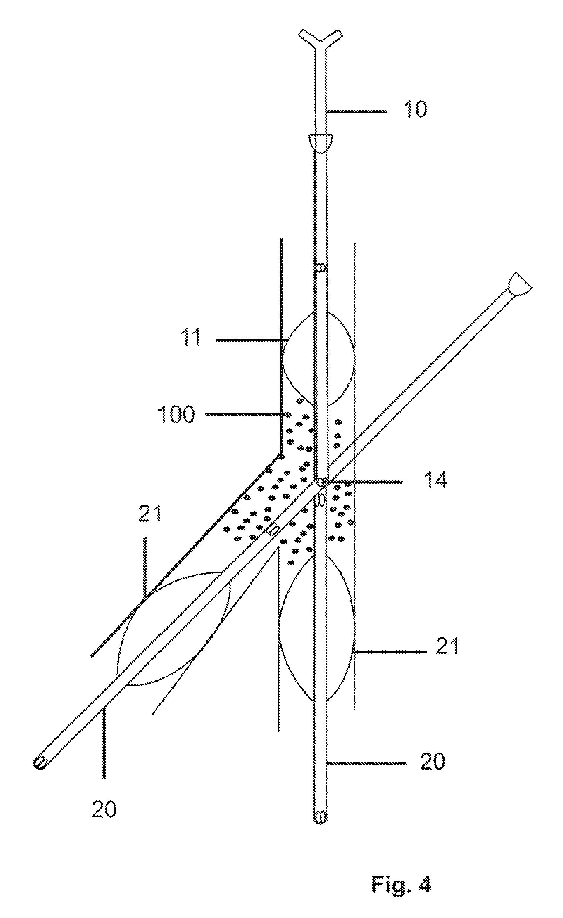

As shown in Figure 4, in such situations as prevention and treatment of restenosis in coronary and peripheral arteries in bifurcations, two occlusion catheters (20) are inserted, one occlusion catheter (20) being inserted in the main vessel and another occlusion catheter (2) in the lateral branch of the artery. Then, the balloon (11) of the occlusion-infusion catheter (10) is inflated, closing the path (100) and avoiding the blood flow passage. Next, the drug is infused. During the infusion of the drug, the occlusion catheters (20) are inflated in order to retain the drug inside the arterial portion (100) to be treated and in such a manner as to create a compartment. The drug is released through the perforations (14) disposed on the distal surface of the occlusion-infusion catheter (10), the drug being limited to the area delimited by the balloons (11 and 21) of the catheters (10 and 20) and penetrating the artery or vessel wall (100) without diffusing it into the blood circulation. After the procedure has been finished, the balloons (11 and 21) are deflated and the catheters (10 and 20) are removed.

## Claims

1. A CATHETER SYSTEM FOR PREVENTING AND TREATING RESTENOSIS IN CORONARY AND PERIPHERAL ARTERIES, IN A SINGLE VESSEL OR IN A BIFURCATION **characterized by** comprising:
a) at least, one occlusion-infusion catheter (10) fitted with a balloon (11) disposed in the distal portion and inflatable through a lumen (12) disposed in the proximal portion of said occlusion-infusion catheter (10), the surface of said occlusion-infusion catheter (10) having perforations intended for inserting and sliding out the guide-wire (13), and one or more perforations (14) on the distal end of the balloon (11) to release the drug;
b) at least, one occlusion catheter (20) fitted with a balloon (21) disposed in the distal portion and inflatable through a lumen (22) disposed in the proximal portion of said occlusion catheter (20), and perforations on the surface intended for inserting and sliding out the guide-wire (23).

2. A CATHETER SYSTEM FOR PREVENTING AND TREATING RESTENOSIS IN CORONARY AND PERIPHERAL ARTERIES IN A SINGLE VESSEL OR IN A BIFURCATION, as claimed in 01, **characterized by** the fact that the balloon (11) of the occlusion-infusion catheter (10) is preferably complacent.

3. A CATHETER SYSTEM FOR PREVENTING AND TREATING RESTENOSIS IN CORONARY AND PERIPHERAL ARTERIES IN A SINGLE VESSEL OR IN A BIFURCATION, as claimed in 01, **characterized by** the fact that the balloon (21) of the occlusion catheter (20) is preferably complacent.
